# EUROPEAN PATENT SPECIFICATION

(11) **EP 1 796 650 B1**
(45) Date of publication and mention of the grant of the patent: **07.01.2009**
(21) Application number: 05784613.1
(22) Date of filing: 14.09.2005
(51) Int. Cl.: A61K 9/51

(54) **DELIVERY VEHICLE CONTAINING NANOPARTICLES**
ABGABEVEHIKEL MIT NANOTEILCHEN
VEHICULE D'ADMINISTRATION DE MEDICAMENTS CONTENANT DES NANOPARTICULES

(30) Priority: 14.09.2004 EP 04021855; 25.11.2004 EP 04027997
(43) Date of publication of application: 20.06.2007
(73) Proprietor: NanoDel Technologies GmbH, 39120 Magdeburg (DE)
(72) Inventor: RINGE, Kerstin, 39122 Magdeburg (DE); RADUNZ, Hans-Eckart, 64367 Mühltal (DE); LANDFESTER, Katharina, 89075 Ulm (DE); WEISS, Clemens, 89134 Blaustein (DE)
(74) Representative: Sandmann, Wolfgang
(86) International application number: PCT/EP2005/009894
(87) International publication number: WO 2006/029845

(56) References cited:
- EP-A- 1 010 435
- WO-A-95/22963
- WO-A-02/076441
- WO-A-20/04017945
- FR-A- 2 841 137
- US-A1- 2002 034 474

## Description

The present invention is directed to a delivery vehicle containing nanoparticles. The present invention is further directed to a pharmaceutical composition, comprising said drug delivery vehicle. Further, the invention pertains to a method for manufacturing of said delivery vehicles as well as to the use of these vehicles in medicine.

While many drugs are able to cross biological barriers like the bbb, others do not pass those barriers efficiently or not at all and are only effective when given directly into the target tissue. Thus, many potentially potent drugs are not useful clinically due to their inability to pass biological barriers like the bbb.

A number of approaches have been described in the prior art to increase drug penetration through the biological barriers.

One approach has been to alter the function of the barrier, for example the bbb itself. For instance, osmotic agents, when given peripherally (such as by intravenous injection), result in the opening of the bbb. Further, some drugs acting on the CNS can change the permeability of the bbb for other substances; cholinomimetic arecolines, for instance, have been reported to induce changes of drug penetration through the bbb [Saija, A., Princi, P., De Pasquale, R., Costa, G., "Arecoline but not haloperidol produces changes in the permeability of the blood-brain barrier in the rat." J. Pharm. Pha. 42:135-138(1990)].

Another approach resides in the modification of the drug molecules themselves. For instance, macromolecules, such as proteins, do not pass the bbb at all. For example, one can first isolate the macromolecule active site, i.e., the portion of the molecule which triggers the biologically desirable event, and then use only this active site. Since size is one of the factors in allowing permeability of the bbb, the reduced size is used in the hope that the smaller molecule can now pass the bbb. Other modifications of macromolecules to attempt passage of the bbb include glycating the proteins, thereby enhancing their permeability of the bbb, or forming a prodrug.

U.S. Patent No. 5,260,308 discusses glycating proteins, while U.S. Patent No. 4,933,324 and WO 89/07938 disclose the formation of a prodrug. These prodrugs are formed from a fatty acid carrier and a neuroactive drug which is unable to pass the bbb on its own. A similar system is disclosed in WO89/07938.

Still another approach is the implantation of controlled release polymers which release the active ingredient from a matrix-system directly into the nervous tissue. However, this approach is invasive and requires surgical intervention if implanted directly into the brain or spinal cord [*see* Sabel et al., U.S. Patent No.4,883,666; and Sabel et al., U.S. Patent Application Serial No. 07/407,930].

To overcome these limitations, another approach has been tried in which drug carrier systems are used such as liposomes, erythrocyte ghosts, antibody-conjugates, and monoclonal antibody conjugates. One of the major problems in targeted drug delivery is the rapid opsonization and uptake of injected carriers by the reticuloendothelial system (RES), especially by the macrophages in the liver and spleen. This obstacle may be partially overcome in the case of liposomes by incorporation of so-called "stealth" lipids, such as phosphatidylinositol, monosialoganglioside, or sulfogalactosylceramide. However, all of these systems lack the versatility to permit a wide-range application in medicine.

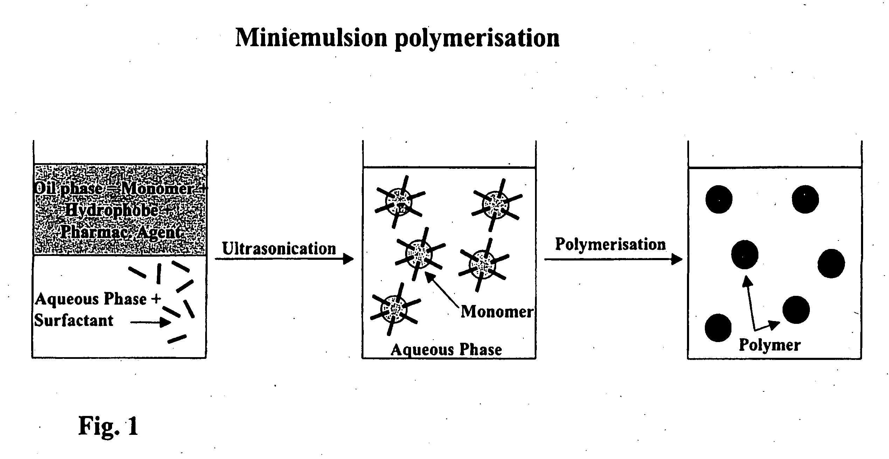

A further approach to carry and direct drugs to a desired target tissue is disclosed in WO 95/022963. Therein, a drug targeting system is disclosed comprising nanoparticles made of a polymeric material, said nanoparticles comprising a drug to be delivered to said mammal and a surfactant coating deposited thereon; and a physiologically acceptable carrier and/or diluent allowing the transport of said nanoparticles to the target within the mammal after administration. In this approach, nanoparticles are used comprising polymeric particles having a diameter of preferably < 1.000 nm. In Fig. 1 b, also nanoparticles are disclosed, which carry the drug encapsulated within a polymeric material, however, the only experiments provided are directed to nanoparticles having a polymeric core particle, on which the drug and subsequently a surfactant coating is applied.

WO 95/022963 does neither contain any information regarding a multilayered polymer coating on a core particle comprising a drug, nor regarding the precise thickness of the coating layer. Furthermore, the nanoparticles used therein are designed as "targeting system" having the object to allow non-penetrating and penetrating drugs to pass the bbb more easily. The term "nanoparticle" as used therein denotes a carrier structure which is sufficiently resistant to chemical and/or physical destruction by the environment of use such that a sufficient amount of the nanoparticles remain substantially intact after injection into the blood stream, or given intraperitoneally or orally, so as to be able to reach the brain at the bbb. WO 95/022963 does not disclose to design and use nanoparticles as a drug delivery vehicle which is a) capable of crossing all major types of biological barriers, for example bbb or mucosa barriers and b) which can achieve a modified release, for example a sustained release or prolonged release, activity in the target tissue after having crossed the biological barrier.

WO 00/041647 relates to a sustained delivery of nucleic acids and other polyionic bioactive agents. In particular, it describes compounds and methods for delivering a polyionic bioactive composition such as a nucleic acid to a tissue of an animal. The compounds include compounds which comprise a matrix comprising the polyionic bioactive agent and wherein at least most of the polyionic bioactive agent at the exterior portion of the matrix is present in a condensed form.

WO 96/20698 provides a sustained release drug delivery system comprising nanoparticles, preferably surface-modified nanoparticles. The nanoparticles have a core of biodegradable, biocompatible polymer or biomaterial. The average diameter of the nanoparticles is typically less than about 300 nm, preferably in the range of 100 nm to 150 nm, and more preferably 10 nm to 50 nm, with a narrow size distribution. The polymeric core may have a bioactive, or bioinactive, agent or combination of agents, incorporated, embedded, entrained, or otherwise made part of the polymer matrix comprising the nanoparticle core. The incorporated bioactive agent is released as the polymer hydrolyzes and dissolves, thereby biodegrading. In WO 96/20698, the sustained release is not achieved by one or more polymeric layers coated on a drug core, but by the degradation of the polymer matrix, in which the drug is distributed. The bioactive agent is added to the polymeric material in forming the nanoparticles.

Therefore, it is an object of the invention to provide a delivery vehicle for a pharmaceutical agent which can be specifically adapted to desired release characteristics. It is in particular an object of the invention to provide a delivery vehicle which is suitable to be used for varying medical conditions and which is able to specifically cross the major physiological or biological barriers of the animal or human body and which subsequently is showing modified release characteristics such as sustained release or prolonged release of the drug in the target tissue. It is a further object of the present invention to provide a method of producing nanoparticles for drug delivery. It is also an object of the present invention to provide an improved method for the manufacture of drug delivery vehicles, which vehicles can be obtained in high yield and solid content. Furthermore, it is an object of the present invention to provide a method for the manufacture of drug delivery vehicles, which is suitable for the large-scale production of said vehicles.

These objects are achieved by the subject-matter of the independent claims. Preferred embodiments are set forth in the dependent claims.

The term "nanoparticles" as used herein generally denotes a carrier structure which is biocompatible and sufficiently resistant to chemical and/or physical destruction by the environment of use such that a sufficient amount of the nanoparticles remain substantially intact after entry into the human or animal body following intraperitoneal or oral or intravenous administration so as to be able to reach the desired target organ or tissue, e. g. the brain, the liver, the kidneys, the lungs etc.

According to a preferred embodiment, said nanoparticles have a diameter of between 1 nm and 20 µm, preferably between 10 nm and 10 µm and most preferably between 100 nm and 1,000 nm.

Furthermore, the nanoparticles as defined above preferably comprise a surfactant coating deposited thereon. A treatment of the nanoparticles with a sufficient coating of an appropriate surfactant allows the adsorbed drug to better traverse physiological barriers as the bbb. Reference is made to several documents disclosing this effect, in particular to WO 95/22963, which is incorporated herein in its entirety.

The biodegradable polymeric material preferably comprises solid or film forming polymers, preferably alkylcyanoacrylates, more preferably butylcyanoacrylates, polylactic acid and polybutyric acid and mixtures and derivatives thereof.

As used herein, the terms "biodegradable polymer" denotes any synthetic or naturally-derived polymeric material which is known as being suitable for uses in the body of a living being, i.e., is biologically inert and physiologically acceptable, non-toxic, and, in the delivery systems of the present invention, is biodegradable in the environment of use, i.e., can be resorbed by the body.

Illustrative biodegradable materials suitable for use in the practice of the invention include naturally derived polymers, such as acacia, gelatin, dextrans, albumins, alginates/starch, and the like; or synthetic polymers, whether hydrophilic or hydrophobic.

Biodegradable synthetic polymers which may be also used to formulate nanoparticles include, but are not limited to, polyesters, such as polyglycolides and polylactic polyglycolic copolymers (PLGA); polyethers, such as such as hydroxy-terminated poly(ε-caprolactone)-polyether or polycaprolactone (PCL); polyanhydrides; polyacrylamides; poly(orthoesters); polyamino acids; and biodegradable polyurethanes. It is to be understood that the term polymer is to be construed to include copolymers and oligomers.

The term "pharmaceutical agents" as used herein comprises both, therapeutic agents and diagnostic agents. It is noted that the terms "drug" and "therapeutic agent" are used interchangeably herein.

The therapeutic agent is preferably selected from substances which are incapable or not sufficiently capable of crossing physiological barrieres without a delivery vehicle or carrier. This physiological barrier is preferably selected from, although not restricted to, the group consisting of blood-brain barrier (bbb), blood-air barrier, blood-cerebrospinal fluid barrier and buccal mucosa. Additionally, the one or more therapeutic agents may have central nervous system activity but cannot cross the blood brain barrier without a delivery vehicle.

According to a preferred embodiment, the delivery vehicle of the invention comprises one or more therapeutic agents selected from the group consisting of drugs acting at synaptic sites and neuroeffector junctional sites; general and local analgetics; hypnotics and sedatives; drugs for the treatment of psychiatric disorders such as depression and schizophrenia; anti-epileptics and anticonvulsants; drugs for the treatment of Parkinson's and Huntington's disease, aging and Alzheimer's disease; anti-obesity drugs; excitatory amino acid antagonists, neurotrophic factors and neuroregenerative agents; trophic factors; drugs aimed at the treatment of CNS trauma or stroke; drugs for the treatment of addiction and drug abuse; antacoids and anti-inflammatory drugs; chemotherapeutic agents for parasitic infections and diseases caused by microbes; immunosuppressive agents and anti-cancer drugs; vitamines; hormones and hormone antagonists; heavy metals and heavy metal antagonists; antagonists for non-metallic toxic agents; cytostatic agents for the treatment of cancer; diagnostic substances for use in nuclear medicine; immunoactive and immunoreactive agents; transmitters and their respective receptor agonists and receptor antagonists, their respective precursors and metabolites; transporter inhibitors; antibiotics; antispasmodics; antihistamines; antinauseants; relaxants; stimulants; sense and antisense oligonucleotides; cerebral dilators; psychotropics; antimanics; vascular dilators and constrictors; anti-hypertensives; drugs for migraine treatment; hypnotics, hyperglycemic and hypoglycemic agents; anti-asthmatics; antiviral agents, preferably anti HIV agents; genetic material suitable for the DNA or anti-sense treatment of diseases; and mixtures thereof.

Typical active ingredients (e.g., drugs) can be any substance affecting the nervous system or used for diagnostic tests of the nervous system. These are described by Gilman et al. (1990), "Goodman and Gilman's - The Pharmacological Basis of Therapeutics", Pergamon Press, New York, and include the following agents:
acetylcholine and synthetic choline esters, naturally occurring cholinomimetic alkaloids and their synthetic congeners, anticholinesterase agents, ganglionic stimulants, atropine, scopolamine and related antimuscarinic drugs, catecholamines and sympathomimetic drugs, such as epinephrine, norepinephrine and dopamine, adrenergic agonists, adrenergic receptor antagonists, transmitters such as GABA, glycine, glutamate, acetylcholine, dopamine, 5-hydroxytryptamine, and histamine, neuroactive peptides; analgesics and anesthetics such as opioid analgesics and antagonists; preanesthetic and anesthetic medications such as benzodiazepines, barbiturates, antihistamines, phenothiazines and butylphenones; opioids; antiemetics; anticholinergic drugs such as atropine, scopolamine or glycopyrrolate; cocaine; chloral derivatives; ethchlorvynol; glutethimide; methyprylon; meprobamate; paraldehyde; disulfiram; morphine, fentanyl and naloxone; psychiatric drugs such as phenothiazines, thioxanthenes and other heterocyclic compounds (e.g., halperiodol); tricyclic antidepressants such as desimipramine and imipramine; atypical antidepressants (e.g., fluoxetine and trazodone), monoamine oxidase inhibitors such as isocarboxazid; lithium salts; anxiolytics such as chlordiazepoxyd and diazepam; anti-epileptics including hydantoins, anticonvulsant barbiturates, iminostilbines (such as carbamazepine), succinimides, valproic acid, oxazolidinediones and benzodiazepines. Anti-Parkinson drugs such as L-DOPA/CARBIDOPA, apomorphine, amatadine, ergolines, selegeline, ropinorole, bromocriptine mesylate and anticholinergic agents;
antispasticity agents such as baclofen, diazepam and dantrolene; neuroprotective agents, such as excitatory amino acid antagonists, neurotrophic factors and brain derived neurotrophic factor, ciliary neurotrophic factor, or nerve growth factor; neurotrophine (NT) 3 (NT3); NT4 and NT5; gangliosides; neuroregenerative agents; drugs for the treatment of addiction and drug abuse include opioid antagonists and anti-depressants; autocoids and anti-inflammatory drugs such as histamine, bradykinin, kailidin and their respective agonists and antagonists; chemotherapeutic agents for parasitic infections and microbial diseases; anti-cancer drugs including alkylating agents (e.g., nitrosoureas) and antimetabolites; nitrogen mustards, ethylenamines and methylmelamines; alkylsulfonates; folic acid analogs; pyrimidine analogs, purine analogs, vinca alkaloids; and antibiotics.

According to a preferred embodiment, the diagnostic agent is selected from the group consisting of diagnostics useful in the diagnosis in nuclear medicine and in radiation therapy.

The surfactant coating and/or the stabilizer preferably comprises one or more of the following substances:
fatty acid esters of glycerols, sorbitol and other multifunctional alcohols, preferably, glycerol monostearate, sorbitan monolaurate, or sorbitan monoleate; polysorbates, preferably polysorbate 60 and most preferably polysorbate 80; poloxamers, preferably poloxamer 188, 338 or 407; poloxamines, preferably poloxamine 904, 908 or 1508; polyoxyethylene ethers and polyoxyethylene esters; ethoxylated triglycerides; ethoxylated phenols and ethoxylated diphenols; surfactants of the Genapol TM and Bauki series; metal salts of fatty acids, metal salts of fatty alcohol sulfates, sodium lauryl sulfate; and metal salts of sulfosuccinates; preferably polyoxyethylene glycols, more preferably Lutensol 50 or 80; sodium dodecyl sulfate; and mixtures of two or more of said substances.

As indicated above, polysorbate 80 is most preferred.

According to a further aspect, the present invention is directed to a pharmaceutical composition, comprising the delivery vehicle as defined herein and a pharmaceutically acceptable carrier and/or diluent.

In order to use the nanoparticles in a practical embodiment, they may be reconstituted into a suspension with distilled water or normal saline at physiological pH and osmolarity.

Typically, the nanoparticles are present in the injectable suspension at a concentration ranging from 0.1 mg nanoparticles per ml suspending fluid to 100 mg nanoparticles per ml suspending fluid. 10 mg nanoparticles per ml is preferred. The amount of nanoparticles used will strongly depend on the amount of pharmaceutical agent contained in an individual nanoparticle and the skilled artisan or the physician in charge will be readily able to adapt the dosage of the nanoparticles to the specific circumstances.

Preferably, the delivery vehicle or the pharmaceutical composition are showing a prolonged release or sustained release of said one or more pharmaceutical agents *in vivo.* Those nanoparticles made in accordance with the principles of the invention biodegrade in periods of time ranging from a few hours to 6 months or more.

Alternatively, the pharmaceutical composition may take other forms required to transfer the delivery vehicle of the invention to and across other physiological barriers, for example to and across the blood-air barrier. Then it may, for example have the form of an aerosol or the like in order to deliver the composition by inhalation to the barrier in question.

According to an aspect, the present invention provides a method for the manufacture of a delivery vehicle for a pharmaceutical agent, comprising the steps of:
a) preparing particles of one or more pharmaceutical agents;
b) preparing a suspension of said particles in a suitable solvent;
c) adding said suspension to a mixture containing a liquid and monomers of one or more biodegradable polymeric materials and polymerizing said monomers in order to provide a polymeric coating on said particles, and isolating said nanoparticles;

As an alternative, in step b) also a solution of said agents in a suitable solvent is used or the one or more pharmaceutical agents are provided in pure form. In the subsequent step c), the solution of said agents or the one or more pharmaceutical agents in pure form are added to a mixture containing a liquid and monomers of one or more biodegradable polymeric materials and polymerizing said monomers in order to provide nanoparticles.

Basically, it is noted that if in step b) a suspension is prepared, a system containing solid and fluid components is established. It is then essential that the pharmaceutical agent remains unsolved in that suspension. Thus, whenever a hydrophilic pharmaceutical agent is used, it should be suspended in a lipohilic solvent or vice versa. In case of a suspension, the solid component preferably has the form of a crystal, in particular of a nanocrystal.

As an alternative, the one or more pharmaceutical agents may be used in pure form in step c), for example in their natural state (fluid or solid, e.g. an oil or a powder). In this case, the method of the present invention will lead to nanoparticles, in which the one or more pharmaceutical agents are dispersed throughout the nanoparticles thus leading to a polymeric matrix by which the one or more pharmaceutical agents are released *in vivo* in a controlled way.

The present invention for the first time presents a method of forming pharmaceutical agent containing nanoparticles, wherein the pharmaceutical agents are contacted and mixed with the monomers before the polymerisation step is performed. Therefore, the one or more pharmaceutical agents may be incorporated in a different way then in the prior art approaches, in which the agent usually is coated on the nanoparticles subsequent to the polymerisation step (thus not incorporating same into the particles).

The method of polymerisation is the miniemulsion technique as developed by K. Landfester et al.

Miniemulsions are dispersions of critically stabilized oil droplets with a size between 50 and 500 nm prepared by shearing a system containing oil, water, a surfactant and a hydrophobe. Polymerizations in such miniemulsions result in particles which have about the same size as the initial droplets. The principle of miniemulsion polymerization is schematically shown in Figure 1 ( K. Landfester, Polyreactions in miniemulsions, Macromol. Rapid Comm. 2001, 896-936. K. Landfester, N. Bechthold, F. Tiarks, and M. Antonietti, Formulation and stability mechanisms of polymerizable miniemulsions. Macromolecules 1999, 32, 5222. K. Landfester, Recent Developments in Miniemulsions - Fonnation and Stability Mechanisms. Macromol. Symp. 2000, 150, 171).

The nanoparticles in the prior art were prepared for example by what is generically termed as an "in-solvent emulsification-evaporation technique" using single (oil-in-water) or multiply emulsifications (water-in-oil-in-water) depending upon whether the incorporated pharmaceutical agent is hydrophobic or hydrophilic (as explained above).

After forming the polymeric particles containing a pharmaceutical agent, the solvent is evaporated from the emulsion. The nanoparticles are separated from the remainder by centrifugation, or preferably ultracentrifugation (120,000 to 145,000 g), washed with water, and re-centrifuged and decanted. Organic solvents which remain in the nanoparticles formed, however, may cause problems in subsequent *in vivo* applications (what is not the case in the miniemulsion method explained above).

Therefore, the present invention is also and preferably directed to a method of preparing nanoparticles containing the steps of:
- providing a reaction system comprising O and W type liquid phases, a stabilizer, one or more pharmaceutical agents and polymerizable monomers,
- forming an O/W type miniemulsion, and
- polymerizing said monomers in order to form nanoparticles.

As a general remark, the nanoparticles of the present invention are formed by a "miniemulsion method", which is per se well known and is, for example, disclosed in K. Landfester, "Polyreactions in miniemulsions", Macromol. Rapid Comm. 2001, 896-936. K. Landfester, N. Bechthold, F. Tiarks, and M. Antonietti, "Formulation and stability mechanisms of polymerizable miniemulsions". This technology basically differs from the well known conventional methods by involving a different order of manufacturing steps: in this approach, a miniemulsion, containing dispersed hydrophobic, monomer containing droplets in a hydrophilic continous phase, are formed to polymeric particles. In the conventional approach, the polymers are directly formed from the solution containing monomers.

Thus, the term "miniemulsion" as used herein generally means a technology, by which a dispersion is made containing a continuous W (hydrophilic) and an O (hydrophobic) phase, dispersed in the continuous phase. Further, this term is directed to emulsion droplets formed of 1 to 1.000 nm, usually approximately between 50 and 500 nm (the size of the particles following polymerization is identical or almost identical).

One of the major drawbacks of the conventional approach can be seen in the fact that only suspensions containing about I % of nanoparticles (solid content) may be obtained therewith. In contrast, the present miniemulsion approach allows a solid content of nanoparticles of even 15-24% or more based on a monomer content of 25% w/w (based on the overall weight of the O/W reaction system). Thus, the yield of solid nanoparticles is dramatically increased and corresponds to about 60-96% of the employed monomers.

With this method, it is possible to obtain nanoparticle suspensions of high solid content (range: 15%-24%) and thus in a high yield (60-96%), which is very important in terms of up-scaling. The process is reproducible. The mean diameter of the generated nanoparticles is 200-300 nm (range < 1 µm).

As an example, if the O phase is consisting of 6 g monomer + 250 mg hydrophobe, the W phase is 24 g 0.01 N HCl + stabilizer, the maximum content of solids will be 25% w/w (6/24 x 100) corresponding to a yield of 100%. A content of 20% w/w then corresponds to a yield of 80%. In this case, the remaining 20% are represented by monomers, which were lost during polymerization, for example by polymerizing at the vessel surface or at the homogenizer or the like.

Preferably, the one or more pharmaceutical agents and the monomers are contained in the O phase. In this case, the pharmaceutical agents are finely distributed into the nanoparticles formed by the polymerisation step. As an alternative, the one or more pharmaceutical agents are present in the W phase and the monomers are contained in the O phase. It turned out that also in this case, a remarkable amount of the pharmaceutical agent is incorporated into the nanoparticles formed. We do not want to be bound to any specific theory, however, it is assumed that the pharmaceutical agents contained in the W phase are intimately contacted with the O phase by preparing the miniemulsion, for example by applying high shear forces, leading to a distribution of the agent also in the O phase.

Additionally, solution mediators may be used to bring agents into solution, which under normal circumstances would not dissolve in a sufficient amount in the solvent. Examples for this group of substances are dimethylsulfoxide, dimethylformamide, polysorbate 80, vitamine E, polyethylenglycole (PEG 400), Cremophor, or glycerol.

It is furthermore possible to add the one or more pharmaceutical agents to both, the O and the W phase, if this is desired.

In a preferred embodiment, the stabilizer used is contained in the W phase.

As mentioned above, the already known miniemulsion technique as, for example, disclosed in Landfester et al. (supra), may be used to prepare the miniemulsion. This may, for example be done by at first combining monomers in an O phase and a W phase and a stabilizer (defined below). It is noted that the W phase may contain further ingredients, for example HCl for setting a suitable pH value. Second, the reaction system may be mixed by, for example, a homogenizer or the like, in order to mix all ingredients.

The preparation of the miniemulsion itself is performed by applying high shear forces to the reaction system, for example by ultrasound and high pressure homogenizers. Furthermore, the shear forces may be applied for a time range of from 1-5 min. depending on the size of the reaction system and the homogenizer used. Basically, a time range of between 2 and 4 min. is regarded as being sufficient.

The ultrasound homogenizer may have an amplitude of about 60-100%, preferably about 70%.

According to an embodiment, the temperatures used in this process are preferably from -1 to 5 °C, preferably 0°C.

Generally the weight ratio of O to W phase is from 15-40 % w/w, preferably 20-30 % w/w and more preferably about 25 % w/w.

Following preparing the miniemulsion, the polymerisation process is started, for example by simply increasing the pH to a value of pH 7,0 or the like. This may be done, whenever PBCA monomers are used in the reaction system and the pH increase is performed, for example, by adding a sufficient amount of K₂CO₃ to the reaction system or by pouring the prepared miniemulsion into an aqueous solution of NaOH.

According to a further embodiment, the O phase is containing a hydrophobe, preferably selected from olive oil, miglyol and/or hexadecane. The amount of hydrophobe is usually relatively small and should be sufficient to prevent Ostwald ripening (about 2-10% w/w based on the overall weight of the O-phase (further containing the monomer)).

Preferably, the particles in step a) are prepared by spray drying a solution containing the one or more pharmaceutical agents. Alternatively, the particles in step a) may be prepared by grinding and optionally sieving or by high-pressure homogenization of the one or more pharmaceutical agents. The liquid used in step c) preferably comprises a lipophilic solvent, preferably n-hexane, hexadecane or miglyol.

In a preferred embodiment, the method of the present invention contains an additional step, wherein the stabilizer is at least partially removed from the obtained nanoparticles. This can preferably be done by dialysis or centrifugation. It surprisingly turned out that, although required in the method of manufacture per se, the stabilizers may be at least partially removed after the final nanoparticles were formed. In other words, an "excess" of stabilizers can be removed which is not required for maintaining the stability of the nanoparticles, but can cause potential risks for *in vivo* applications. It is assumed that the lowest possible amount of stabilizers in the nanoparticles should be regarded as having the lowest *in vivo* risk.

According to a further aspect, the present invention is directed to a delivery vehicle, which is obtainable by the method as defined above.

It is noted that, although the present disclosure is showing some embodiments only in connection with the method or the delivery vehicle/nanoparticles etc., their contents also extend to all respective other aspects contained in this application.

The present invention further provides the use of the delivery vehicle or a pharmaceutical composition as defined herein for the manufacture of a medicament for the treatment of diseases and conditions, requiring a pharmaceutical agent to cross one or more physiological barrieres. It is in particular used for the treatment of diseases corresponding to the drugs listed hereinabove.

In particular, the delivery vehicle will find application in the treatment of diseases related to the CNS. Furthermore, this includes the treatment of AIDS, since in many people with advanced AIDS - possibly a third of adults and half of all children - HIV also infiltrates and harms the brain, triggering HIV-associated dementia. The disorder is marked by poor concentration, decreased memory and slow thinking and movements. However, it is particularly hard to target the virus in the brain. Here, the present invention may open up new therapeutic successes by delivering anti-HIV agents to the brain.

The invention is now further illustrated by the accompanying drawings, in which:
Fig. 1 is an illustration showing the miniemulsion polymerzation technique;

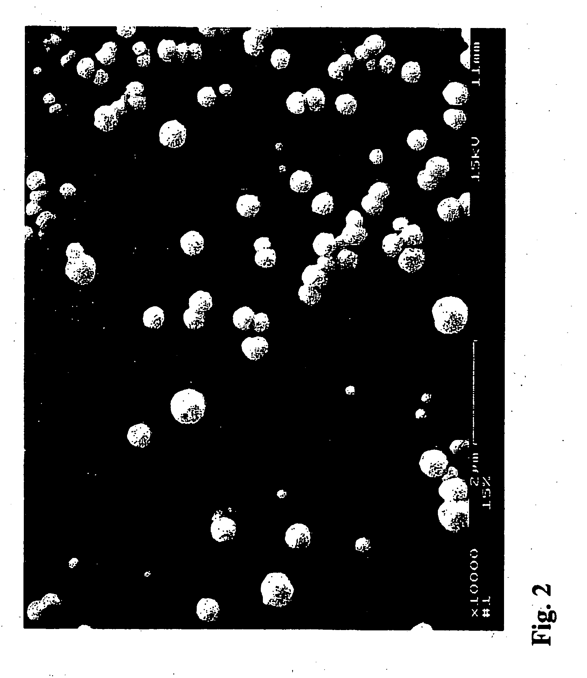

Fig. 2 is a photograph of nanoparticles manufactured in accordance with the present invention;

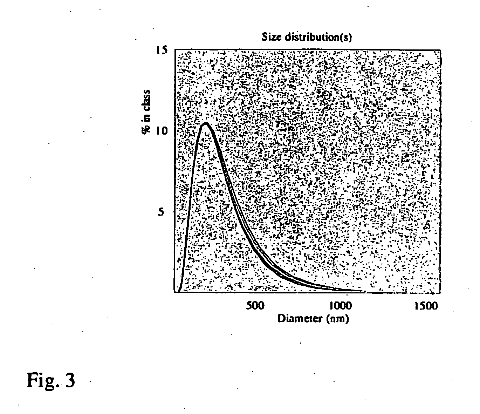

Fig. 3 is a representation of the particle size distribution of nanoparticles manufactured in eccordance with present invention.

### Examples:

### 1. Encapsulation of Paclitaxel (in DMSO) by n-Butylcyanacrylat using the Miniemulsion Method

Two separate solutions are prepared.
Solution 1: 2,4 ml of Hydrochloric acid (0,1 mol·l⁻¹) are added into a 1,5 ml flask (Eppendorf Tube). Then 0,06 g sodium dodecylsulfate (SDS) are added to this solution. The resulting solution is stirred until the SDS is completely dissolved.
Solution 2: a) 30 mg of Paclitaxel and 30 µl of Dimethylsulfoxide (DMSO) are added in a 5 ml flask to prepare a solution of Paclitaxel in DMSO. b) Then 0,0323 mL (0,025 mg) Hexadecane and at last 0,5455 mL (0,6 g) *n*-Butyl-α-cyanacrylate (lndermil) are added into a 5 ml flask. The flask is pivoted until a homogeneous solution is formed. At last solution a) and b) are mixed to prepare a homogeneous solution.

Solution 2 is added to solution 1 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 2,4 ml of 0,1 mol 1⁻¹ aqueous NaOH solution (10 ml flask). The obtained suspension is then stirred for 5 min. The nanoparticle suspension possesses a mean particle size (photon correlation spectroscopy) of 270,2 ± 2,3 nm.

For determining the amount of encapsulated Paclitaxel, I ml of the formed nanoparticle suspension is centrifuged for 30 min. at 20.000 rpm to remove possible unbound Paclitaxel. The amount of Paclitaxel in the supernatant is determined by uv-spectroscopy. The amount of encapsulated Paclitaxel is determined by using uv-spectroscopy. Therefore the pellet is dissolved in 1 ml Ethylacetate. The total amount of Paclitaxel detected by these methods could be determined to be 70,2% (0,35 mg related to 10 mg NP), whereas the amount of encapsulated Paclitaxel was found to be 24,4% (0,12 mg/10 mg NP) and the amount of free Paclitaxel was found to be 45,8% (0,23 mg related to 10 mg/ml nanoparticles).

### 2. Encapsulation of Paclitaxel by n-Butylcyanacrylat using the Miniemulsion Method

Two separate solutions are prepared.
Solution 1: 2,4 ml of Hydrochloric acid (0,1 mol·l⁻¹) are added into a 1,5 ml flask (Eppendorf Tube). Then 0,06 g sodium dodecylsulfate (SDS) are added to this solution. The resulting solution is stirred until the SDS is completely dissolved.
Solution 2: 30 mg of Paclitaxel, then 0,0323 mL (0,025 mg) Hexadecane and at last 0,5455 mL (0,6 g) *n*-Butyl-α-cyanacrylate (Indermil) are added into a 5 ml flask. The flask is pivoted until a homogeneous suspension is formed.

Solution 2 is added to solution 1 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 2,4 ml of 0,1 mol l⁻¹ aqueous NaOH solution (10 ml flask). The obtained suspension is then stirred for 5 min. The nanoparticle suspension possesses a mean particle size (photon correlation spectroscopy) of 270,2 ± 2,3 nm.

For determining the amount of encapsulated Paclitaxel, 1 ml of the formed nanoparticle suspension is centrifuged for 30 min. at 20.000 rpm to remove possible unbound Paclitaxel. The amount of Paclitaxel in the supernatant is determined by uv-spectroscopy. The amount of encapsulated Paclitaxel is determined by using uv-spectroscopy. Therefore the pellet is dissolved in 1 ml Ethylacetate. The total amount of Paclitaxel detected by these methods could be determined to be 99% (0,49 mg related to 10 mg NP), whereas the amount of encapsulated Paclitaxel was found to be 76% (0,38 mg/10 mg NP) and the amount of free Paclitaxel was found to be 23% (0,11 mg related to 10 mg/ml nanoparticles).

### 3. Encapsulation of Vitamin C by n-Butylcyanacrylat using the Miniemulsion Method

Two separate solutions are prepared.
Solution 1: 36,0 ml of Hydrochloric acid (0,1 mol·l⁻¹) are added into a 100 ml flask (PP). Then 0,900 g sodium dodecylsulfate (SDS) and 1,6 g Vitamin C are added to this solution. The resulting solution is stirred until the SDS is completely dissolved.
Solution 2: 0,485 mL (0,375 g) Hexadecane and at last 8,182 mL (9,0 g) *n*-Butyl-α-cyanacrylate (Indermil) are added into a 50 ml flask. The flask is pivoted until a homogeneous solution is formed.

Solution 2 is added to solution 1 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 36,0 ml of 0,1 mol l⁻¹ aqueous NaOH solution (200 ml flask). The obtained suspension is then stirred for 5 min., while adding additional 134,67 ml 0,1 mol⁻¹ NaOH for neutralizing Vitamin C. The nanoparticle suspension possesses a solid content of 47,9 mg/ml and a mean particle size (photon correlation spectroscopy) of 286,7±7,9 nm.

For determining the amount of encapsulated Vitamin C, 1 ml of the formed nanoparticle suspension is centrifuged for 30 min. at 20.000 rpm to remove possible unbound Vitamin C. The amount of free Vitamin C in the supernatant is determined by uv-spectroscopy and could be determined to be 67,4% (1,2 mg related to 10 mg/ml nanoparticles). The amount of encapsulated Vitamin C was found to be 32,6% (0,58 mg/10 mg NP).

### 4. Encapsulation of Vitamin E by n-Butylcyanacrylat using the Miniemulsion Method

Two separate solutions are prepared.
Solution 1: 36,0 ml of Hydrochloric acid (0,1 mol·l⁻¹) are added into a 100 ml flask (PP). Then 0,900 g sodium dodecylsulfate (SDS) are added to this solution. The resulting solution is stirred until the SDS is completely dissolved.
Solution 2: 2,023 g of Vitamin E, then 0,485 mL (0,375 g) Hexadecane and at last 8,182 mL (9,0 g) *n*-Butyl-α-cyanacrylate (Indermil) are added into a 50 ml flask. The flask is pivoted until a homogeneous solution is formed.

Solution 2 is added to solution 1 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 38,0 ml of 0,1 mol l⁻¹ aqueous NaOH solution (100 ml flask). The obtained suspension is then stirred for 5 min. The nanoparticle suspension possesses a solid content of 135,4 mg/ml and a mean particle size (photon correlation spectroscopy) of 125,9±1,1 nm.

For determining the amount of encapsulated Vitamin E, 1 ml of the formed nanoparticle suspension (13,54% (w/w)) is diluted to 1% (w/w). Then 1 ml of the resulting suspension is centrifuged for 30 min. at 20.000 rpm to remove possible unbound Vitamin E. The amount of free Vitamin E in the supernatant is determined by uv-spectroscopy. The amount of encapsulated Vitamin E is determined by using uv-spectroscopy. Therefore the pellet is dissolved in 1 ml Ethylacetate. The total amount detected by these methods could be determined to be 87% (1,60 mg related to 10 mg NP), whereas the amount of encapsulated Vitamin E was found to be 33,5 % (0,616 mg/10 mg NP) and the amount of free Vitamin E was found to be 53,5% (0,98 mg related to 10 mg/ml nanoparticles).

### 5. Encapsulation of nanocrystalline "Compound A" by n-Butylcyanacrylat using the Miniemulsion Method

Compound A is a hydrophobic pharmeutical compound.

Two separate solutions are prepared.
Solution 1: A solution of sodium dodecylsulfate (SDS) in Hydrochloric acid (0,1 mol·l⁻¹) is formed.
Nanocrystals: "Compound A" is dispersed in a 1% (w/w) aqueous polysorbate 80 solution using ultrasonication (1 min.). The nanocrystals of "Compound A" are obtained via high-pressure homogenisation by applying an appropriate amount of cycles at increasing pressures. The resulting nanocrystals can be purified via dialysis.
Solution 2: Nanocrystalline "Compound A", either purified or not purified, Hexadecane and *n*-Butyl-α-cyanacrylate (Indermil) are added into a flask. The flask is pivoted until a homogeneous suspension is formed.

Solution 1 is added to solution 2 and the miniemulsion is immediately formed by ultrasonication (amplitude 70%, 0°C) of the resulting suspension for 2 min. Then the polymerisation of the monomer droplets is initiated by rapidly changing the pH value from 1 to 7 by adding the miniemulsion into 0,1 mol l⁻¹ aqueous NaOH solution. The obtained suspension is then stirred for 5 min. The solid content of the formed nanoparticle suspension is determined gravimetrically and the mean particle size is determined using photon correlation spectroscopy.

For determining the amount of encapsulated "Compound A", 1 ml of the formed nanoparticle suspension is centrifuged for 30 min. at 20.000 rpm to remove possible unbound "Compound A". The amount of free "Compound A" in the supernatant is determined by uv-spectroscopy. The amount of encapsulated "Compound A" is determined by using uv-spectroscopy. Therefore the pellet is dissolved in I ml ethylacetate.

## Claims

1. A method of producing nanoparticles for drug delivery comprising the steps of:
a) providing one or more pharmaceutical agents;
b) preparing a suspension or solution of said agents in a suitable solvent or providing the one or more pharmaceutical agents in pure form;
c) adding said suspension or solution of said agents or the one or more pharmaceutical agents in pure form to a mixture containing a liquid and monomers of one or more biodegradable polymeric materials and polymerizing said monomers in order to provide nanoparticles and isolating same,
**characterized in that** the nanoparticles are prepared by using the steps of:
- providing a reaction system comprising O and W type liquid phases, a stabilizer, one or more pharmaceutical agents and polymerizable monomers,
- forming an O/W type miniemulsion, and
- polymerizing said monomers in order to form nanoparticles.

2. The method of claim 1, wherein the one or more pharmaceutical agents and the monomers are contained in the O phase, or, wherein the one or more pharmaceutical agents are present in the W phase and the monomers are contained in the O phase.

3. The method of claims 1 or 2, wherein the stabilizer is contained in the W phase.

4. The method of one or more of claims 1-3, wherein the pharmaceutical agents in step a) are prepared as particles by spray drying a solution containing the one or more pharmaceutical agents, or, by grinding and optionally sieving the one or more pharmaceutical agents, preferably by high pressure homogenizers.

5. The method of one or more of the preceding claims, wherein the liquid used in step c) or which is contained in the O phase comprises a lipophilic solvent, preferably n-hexane, hexadecane or miglyol.

6. The method of claim 1, wherein the miniemulsion is formed by applying high shear forces to the reaction system, preferably by using ultrasound and/or high pressure homogenizers.

7. The method of one or more of the preceding claims, wherein the biodegradable polymeric material preferably comprises solid or film forming polymers, preferably alkylcyanoacrylates, more preferably butylcyanoacrylates, polylactic acid and polybutyric acid and mixtures and derivatives thereof.

8. The method of one or more of the preceding claims, wherein the one or more pharmaceutical agents are selected from a therapeutic agent and a diagnostic agent, wherein the therapeutic agent is preferably selected from substances which are incapable or not sufficiently capable of crossing physiological barrieres without a delivery vehicle or carrier, wherein the physiological barrier is preferably selected from the group consisting of blood-brain barrier (bbb), blood-air barrier, blood-cerebrospinal fluid barrier and buccal mucosa.

9. The method of claim 8, wherein the one or more therapeutic agents have central nervous system activity but cannot cross or not sufficiently cross the blood brain barrier without a delivery vehicle, and/or are preferably selected from the group consisting of drugs acting at synaptic sites and neuroeffector junctional sites; general and local analgetics; hypnotics and sedatives; drugs for the treatment of psychiatric disorders such as depression and schizophrenia; anti-obesity drugs; anti-epileptics and anticonvulsants; drugs for the treatment of Parkinson's and Huntington's disease, aging and Alzheimer's disease; excitatory amino acid antagonists, neurotrophic factors and neuroregenerative agents; trophic factors; drugs aimed at the treatment of CNS trauma or stroke; drugs for the treatment of addiction and drug abuse; antacoids and anti-inflammatory drugs; chemotherapeutic agents for parasitic infections and diseases caused by microbes; immunosuppressive agents and anti-cancer drugs; vitamins; hormones and hormone antagonists; heavy metals and heavy metal antagonists; antagonists for non-metallic toxic agents; cytostatic agents for the treatment of cancer; diagnostic substances for use in nuclear medicine; immunoactive and immunoreactive agents; transmitters and their respective receptor agonists and receptor antagonists, their respective precursors and metabolites; transporter inhibitors; antibiotics; antispasmodics; antihistamines; antinauseants; relaxants; stimulants; sense and antisense oligonucleotides; cerebral dilators; psychotropics; antimanics; vascular dilators and constrictors; anti-hypertensives; drugs for migraine treatment; hypnotics, hyperglycemic and hypoglycemic agents; anti-asthmatics; antiviral agents, preferably anti HIV agents; genetic material suitable for the DNA or anti-sense treatment of diseases; and mixtures thereof.

10. The method of claim 8 or 9, wherein said diagnostic agent is selected from the group consisting of diagnostics useful in the diagnosis in nuclear medicine and in radiation therapy, and/or, wherein the surfactant coating or the stabilizer comprises one or more of the following substances:
fatty acid esters of glycerols, sorbitol and other multifunctional alcohols, preferably, glycerol monostearate, sorbitan monolaurate, or sorbitan monoleate; poloxamines, preferably poloxamine 904, 908 or 1508; polyoxyethylene ethers and polyoxyethylene esters; ethoxylated triglycerides; ethoxylated phenols and ethoxylated diphenols; surfactants of the Genapol TM and Bauki series; metal salts of fatty acids, metal salts of fatty alcohol sulfates, sodium lauryl sulfate; and metal salts of sulfosuccinates; preferably polysorbates, more preferably polysorbate 60 and most preferably polysorbate 80; preferably poloxamers, more preferably poloxamer 188, 338 or 407; preferably polyoxyethylene glycols, more preferably Lutensol 50 or 80; sodium dodecyl sulfate; and mixtures of two or more of said substances.

11. The method of one or more of the preceding claims, wherein the nanoparticles comprise a surfactant coating deposited thereon.

12. Nanoparticles obtainable by the method of one or more of claims 1-11, or a pharmaceutical composition, comprising said nanoparticle and a pharmaceutically acceptable carrier and/or diluent, wherein the pharmaceutical composition preferably is showing a prolonged release or sustained release of said one or more pharmaceutical agents *in vivo.*

13. Use of the nanoparticles or the pharmaceutical composition of claim 12 for the manufacture of a medicament for the treatment of diseases related to the CNS, for the treatment of AIDS and cancer.

## Patentansprüche

1. Verfahren zur Herstellung von Nanopartikeln zum Arzneistofftransport, umfassend die folgenden Schritte:
a) Bereitstellen eines oder mehrerer pharmazeutischer Mittel;
b) Herstellen einer Suspension oder Lösung dieser Mittel in einem geeigneten Lösungsmittel oder Bereitstellen der ein oder mehreren pharmazeutischen Mittel in reiner Form;
c) Hinzufügen der Suspension oder Lösung der Mittel oder des einen oder der mehreren pharmazeutischen Mittel in reiner Form zu einem Gemisch, das eine Flüssigkeit und Monomere eines oder mehrerer biologisch abbaubarer Polymermaterialien enthält, und Polymerisieren der Monomere, um Nanopartikel bereitzustellen, und Isolieren derselben,
**dadurch gekennzeichnet, dass** die Nanopartikel durch folgende Schritte hergestellt werden:
- Bereitstellen eines Reaktionssystems, das flüssige O- und W-Typ Phasen, einen Stabilisator, ein oder mehrere pharmazeutische Mittel und polymerisierbare Monomere umfasst,
- Ausbilden einer Miniemulsion vom O/W-Typ, und
- Polymerisieren der Monomere, um Nanopartikel zu bilden.

2. Verfahren nach Anspruch 1, wobei die ein oder mehreren pharmazeutischen Mittel und die Monomere in der O-Phase enthalten sind, oder, wobei die ein oder mehreren pharmazeutischen Mittel in der W-Phase vorhanden sind und die Monomere in der O-Phase enthalten sind.

3. Verfahren nach Anspruch 1 oder 2, wobei der Stabilisator in der W-Phase enthalten ist.

4. Verfahren nach einem oder mehreren der Ansprüche 1 bis 3, wobei die pharmazeutischen Mittel in Schritt a) als Teilchen durch Sprühtrocknen einer Lösung hergestellt werden, die ein oder mehrere pharmazeutische Mittel enthält, oder, durch Vermahlen und wahlweise Sieben der ein oder mehreren pharmazeutischen Mittel, vorzugsweise durch Hochdruckhomogenisatoren.

5. Verfahren nach einem oder mehreren der vorhergehenden Ansprüche, wobei die in Schritt c) verwendete oder die in der O-Phase enthaltene Flüssigkeit ein lipophiles Lösungsmittel, vorzugsweise n-Hexan, Hexadecan oder Miglyol, umfasst.

6. Verfahren nach Anspruch 1, wobei die Miniemulsion durch Anwendung hoher Scherkräfte auf das Reaktionssystem gebildet wird, vorzugsweise durch Verwendung von Ultraschall und/oder Hochdruckhomogenisatoren.

7. Verfahren nach einem oder mehreren der vorhergehenden Ansprüche, wobei das biologisch abbaubare Polymermaterial vorzugsweise feste oder filmbildende Polymere umfasst, vorzugsweise Alkylcyanoacrylate, besonders bevorzugt Butylcyanoacrylate, Polymilchsäure und Polybuttersäure und Gemische und Derivate hiervon.

8. Verfahren nach einem oder mehreren der vorhergehenden Ansprüche, wobei die ein oder mehreren pharmazeutischen Mittel aus einem therapeutischen Mittel und einem diagnostischen Mittel ausgewählt sind, wobei das therapeutische Mittel vorzugsweise aus Substanzen ausgewählt ist, die nicht oder nicht ausreichend dazu in der Lage sind, physiologische Barrieren ohne ein Transportmedium oder Träger zu durchqueren, wobei die physiologische Barriere vorzugsweise aus der Gruppe ausgewählt ist, die aus der Blut-Hirn-Schranke (blood-brain barrier = bbb), Blut-Gas-Schranke, Blut-Liquor-Schranke und Wangenschleimhaut besteht.

9. Verfahren nach Anspruch 8, wobei die ein oder mehreren therapeutischen Mittel eine Wirksamkeit auf das Zentralnervensystem aufweisen, jedoch die Blut-Him-Schranke ohne Trägermedium nicht ausreichend durchqueren können und/oder vorzugsweise aus der Gruppe ausgewählt sind, die aus Arzneistoffen, die an synaptischen und Neuroeffektorknotenpunkten wirken; aus allgemeinen oder Lokalanalgetika, Hypnotika und Sedativa; Arzneistoffen zur Behandlung psychiatrischer Störungen wie beispielsweise Depression und Schizophrenie; Arzneistoffen gegen Übergewicht; Antiepileptika und Antikonvulsiva; Arzneistoffen zur Behandlung der Parkinson- und Huntington-Krankheit, des Alterns und der Alzheimer-Krankheit; excitatorischen Aminosäureantagonisten, neurotrophen Faktoren und neuroregenerativen Mitteln; trophischen Faktoren; Arzneistoffen, die die Behandlung von ZNS-Traumata oder Schlaganfällen zum Ziel haben; Arzneistoffen zur Behandlung der Abhängigkeit und des Arzneistoffmissbrauchs; Autacoida und antiinflammatorischen Arzneistoffen; chemotherapeutischen Mitteln gegen Parasiteninfektionen und Erkrankungen, die durch Mikroben verursacht sind; immunsuppressiven Mitteln und Antikrebsmitteln; Vitaminen; Hormonen und Hormonantagonisten; Schwermetallen und Schwermetallantagonisten; Antagonisten für nichtmetallische toxische Mittel; zytostatischen Mitteln zur Behandlung von Krebs; diagnostischen Substanzen zur Verwendung in der Nuklearmedizin; immunaktiven und immunreaktiven Mitteln; Transmittern und ihren jeweiligen Rezeptoragonisten und Rezeptorantagonisten, ihren jeweiligen Vorläufern und Metaboliten; Transporterinhibitoren; Antibiotika; Antispasmodika; Antihistaminika; gegen Nausea wirkenden Mitteln; Relaxantien; Stimulantien; sense- und antisense-Oligonukleotiden; Cerebraldilatatoren; Psychotropika; Antimanika; Gefäßdilatatoren und -konstriktoren; Antihypertensiva; Arzneistoffen zur Migränebehandlung; Hypnotika, hyperglykämischen und hypoglykämischen Mitteln, Antiasthmatika; antiviralen Mitteln, vorzugsweise Anti-HIV-Mitteln; genetischem Material, das für die DNA- oder Antisensebehandlung von Erkrankungen geeignet ist; und Gemischen hiervon besteht.

10. Verfahren nach Anspruch 8 oder 9, wobei das diagnostische Mittel aus der Gruppe ausgewählt ist, die aus Diagnostika besteht, die in der Diagnose in der Nuklearmedizin und der Strahlentherapie nützlich sind und/oder wobei die Beschichtung mit den oberflächenaktiven Mitteln oder der Stabilisator ein oder mehrere der folgenden Substanzen umfasst:
Fettsäureester von Glycerolen, Sorbitol und anderen multifunktionellen Alkoholen, vorzugsweise Glycerolmonostearat, Sorbitanmonolaurat oder Sorbitanmonooleat; Poloxamine, vorzugsweise Poloxamin 904, 908 oder 1508; Polyoxyethylenether und
Polyoxyethylenester; ethoxylierte Triglyzeride; ethoxylierte Phenole und ethoxylierte Diphenole; oberflächenaktive Stoffe der Genapol TM und Bauki-Reihen; Metallsalze von Fettsäuren, Metallsalze von Fettalkoholsulfaten; Natriumlaurylsulfat; und
Metallsalze von Sulfosuccinaten; vorzugsweise Polysorbate, besonders bevorzugt Polysorbat 60 und am meisten bevorzugt Polysorbat 80; vorzugsweise Poloxamere, besonders bevorzugt Poloxamer 188, 338 oder 407; vorzugsweise
Polyoxyethylenglycole, besonders bevorzugt Lutensol 50 oder 80;
Natriumdodecylsulfat; und Gemische aus zwei oder mehreren dieser Substanzen.

11. Verfahren nach einem oder mehreren der vorhergehenden Ansprüche, wobei die Nanopartikel eine Beschichtung aus einem oberflächenaktiven Stoff umfassen, der darauf abgelagert ist.

12. Nanopartikel, die durch das Verfahren nach einem oder mehreren der Ansprüche 1 bis 11 gewinnbar sind, oder pharmazeutische Zusammensetzung, die die Nanopartikel und einen pharmazeutisch verträglichen Träger und/oder ein Verdünnungsmittel umfasst, wobei die pharmazeutische Zusammensetzung vorzugsweise eine verlängerte Freisetzung oder verzögerte Freisetzung eines oder mehrerer pharmazeutischer Mittel *in vivo* zeigt.

13. Verwendung der Nanopartikel oder der pharmazeutischen Zusammensetzung nach Anspruch 12 zur Herstellung eines Medikamentes zur Behandlung von Erkrankungen, die mit dem ZNS in Verbindung stehen, zur Behandlung von AIDS und Krebs.

## Revendications

1. Procédé de production de nanoparticules pour la délivrance de médicament comprenant les étapes consistant à:
a) fournir un ou plusieurs agents pharmaceutiques;
b) préparer une suspension ou une solution desdits agents dans un solvant approprié ou fournir le ou les plusieurs agents pharmaceutiques sous forme pure;
c) ajouter ladite suspension ou solution desdits agents ou le ou les plusieurs agents pharmaceutiques sous forme pure à un mélange contenant un liquide et des monomères d'une ou plusieurs matières polymères biodégradables et polymériser lesdits monomères afin de fournir des nanoparticules et isoler ces dernières,
**caractérisé en ce que** les nanoparticules sont préparées en utilisant les étapes consistant à:
- fournir un système de réaction comprenant des phases liquides de type O et W, un stabilisateur, un ou plusieurs agents pharmaceutiques et des monomères pouvant être polymérisés,
- former une mini-émulsion du type O/W, et
- polymériser lesdits monomères afin de former des nanoparticules.

2. Procédé selon la revendication 1, dans lequel le ou les plusieurs agents pharmaceutiques et les monomères sont contenus dans la phase O, ou, dans lequel le ou les plusieurs agents pharmaceutiques sont présents dans la phase W et les monomères sont contenus dans la phase O.

3. Procédé selon les revendications 1 ou 2, dans lequel le stabilisateur est contenu dans la phase W.

4. Procédé selon une ou plusieurs des revendications 1 à 3, dans lequel les agents pharmaceutiques dans l'étape a) sont préparés comme des particules en séchant par atomisation une solution contenant le ou les plusieurs agents pharmaceutiques, ou, en broyant et en tamisant de manière facultative le ou les plusieurs agents pharmaceutiques, de préférence par des homogénéisateurs à haute pression.

5. Procédé selon une ou plusieurs des revendications précédentes, dans lequel le liquide utilisé dans l'étape c) ou qui est contenu dans la phase O comprend un solvant lipophile, de préférence du n-hexane, de l'hexadécane ou du miglyol.

6. Procédé selon la revendication 1, dans lequel la mini-émulsion est formée en appliquant des forces de cisaillement élevées au système de réaction, de préférence en utilisant des ultrasons et/ou des homogénéisateurs à haute pression.

7. Procédé selon une ou plusieurs des revendications précédentes, dans lequel la matière polymère biodégradable comprend de préférence des polymères solides ou filmogènes, de préférence des alkylcyanoacrylates, encore mieux des cyanoacrylates de butyle, de l'acide polylactique et de l'acide polybutyrique et des mélanges et des dérivés de ces derniers.

8. Procédé selon une ou plusieurs des revendications précédentes, dans lequel le ou les plusieurs agents pharmaceutiques sont sélectionnés parmi un agent thérapeutique et un agent de diagnostic, dans lequel l'agent thérapeutique est de préférence sélectionné parmi des substances qui sont incapables ou insuffisamment capables de traverser des barrières physiologiques sans un véhicule ou un porteur de délivrance, dans lequel la barrière physiologique est de préférence sélectionnée dans le groupe constitué par la barrière hématoencéphalique (bbb), la barrière alvéolo-capillaire, la barrière hématoméningée et la muqueuse buccale.

9. Procédé selon la revendication 8, dans lequel le ou les plusieurs agents thérapeutiques ont une activité de système nerveux central mais ne peuvent pas traverser ou insuffisamment traverser la barrière hématoencéphalique sans un véhicule de délivrance, et/ou sont de préférence sélectionnés dans le groupe constitué par les médicaments agissant au niveau des sites synaptiques et des sites de jonction d'effecteur neurologique; les antalgiques généraux et locaux; les hypnotiques et sédatifs; les médicaments pour le traitement de maladies psychiatriques comme la dépression et la schizophrénie; les médicaments anti-obésité; les antiépileptiques et les anticonvulsivants; les médicaments pour le traitement de la maladie de Parkinson et de Huntington, la vieillesse et la maladie d'Alzheimer; les antagonistes d'acide aminé d'excitation, les facteurs neurotrophiques et les régénérateurs neurologiques; les facteurs trophiques; les médicaments destinés au traitement de trauma du SNC (système nerveux central) ou d'accident vasculaire cérébral; les médicaments pour le traitement de la pharmacodépendance et de la toxicomanie; les médicaments antiacides et anti-inflammatoires; les agents chimiothérapiques pour les infections parasites et les maladies provoquées par des microbes; les immunosuppresseurs et les médicaments anti-cancer; les vitamines; les hormones et les antagonistes des hormones; les métaux lourds et les antagonistes des métaux lourds; les antagonistes pour les agents toxiques non métalliques; les agents cytostatiques pour le traitement du cancer; les substances de diagnostic à utiliser dans la médecine nucléaire; les agents immunoactifs et immunoréactifs; les émetteurs et leurs agonistes récepteurs et antagonistes récepteurs respectifs, leurs précurseurs et métabolites respectifs; les inhibiteurs de transporteur; les antibiotiques; les spasmolytiques; les antihistaminiques; les antinauséeux; les tranquillisants; les stimulants; les oligonucléotides sens et antisens; les dilatateurs cérébraux; les psychotropes; les neuroleptiques antimaniaques; les dilatateurs et constricteurs vasculaires; les antihypertenseurs; les médicaments pour le traitement de la migraine; les hypnotiques, les agents hyperglycémiants et hypoglycémiants; les antiasthmatiques; les agents antiviraux, de préférence les agents anti-HIV; le matériel génétique approprié pour l'ADN ou le traitement antisens de maladies; et des mélanges de ces derniers.

10. Procédé selon la revendication 8 ou 9, dans lequel ledit agent de diagnostic est sélectionné dans le groupe constitué par les diagnostics utiles dans la diagnose en médecine nucléaire et dans la thérapie de radiation, et/ou, dans lequel le revêtement d'agent de surface ou le stabilisateur comprend une ou plusieurs des substances suivantes:
les esters d'acide gras de glycérols, sorbitol et autres alcools multifonctionnels, de préférence, le monostéarate de glycérol, le monolaurate de sorbitane, ou le monooléate de sorbitane; les poloxamines, de préférence la poloxamine 904, 908 ou 1508; les éthers de polyoxyéthylène et les esters de polyoxyéthylène; les triglycérides éthoxylés; les phénols éthoxylés et les diphénols éthoxylés; les agents de surface des séries Genapol™ et Bauki; les sels de métal d'acides gras, les sels de métal de sulfates d'alcool gras, le sulfate de sodium laurique; et les sels de métal de sulfosuccinates; de préférence les polysorbates, mieux le polysorbate 60 et encore mieux le polysorbate 80; de préférence les poloxamères, mieux le poloxamère 188, 338 ou 407; de préférence les glycols de polyoxyéthylène, mieux le Lutensol 50 ou 80; le dodécylsulfate de sodium; et les mélanges de deux ou plusieurs desdites substances.

11. Procédé selon une ou plusieurs des revendications précédentes, dans lequel les nanoparticules comprennent un revêtement d'agent de surface déposé dessus.

12. Nanoparticules pouvant être obtenues par le procédé selon une ou plusieurs des revendications 1 à 11, ou une composition pharmaceutique comprenant ladite nanoparticule et un porteur acceptable d'un point de vue pharmaceutique et/ou un diluant, dans lesquelles la composition pharmaceutique présente de préférence une libération prolongée ou une libération soutenue du ou desdits un ou plusieurs agents pharmaceutiques *in vivo.*

13. Utilisation des nanoparticules ou de la composition pharmaceutique selon la revendication 12 pour la fabrication d'un médicament pour le traitement de maladies liées au SNC, pour le traitement du SIDA et du cancer.
